# EUROPÄISCHE PATENTSCHRIFT

(11) **EP 3 416 594 B1**
(45) Veröffentlichungstag und Bekanntmachung des Hinweises auf die Patenterteilung: **18.03.2020**
(21) Anmeldenummer: 17701880.1
(22) Anmeldetag: 30.01.2017
(51) Int. Cl.: A61F 2/30, A61F 2/32, A61F 2/34, A61F 2/28

(54) **IMPLANTAT ZUR REKONSTRUKTION EINER HÜFTGELENKPFANNE UND ZUMINDEST EINES TEILS EINER BECKENSTRUKTUR**
IMPLANT FOR THE RECONSTRUCTION OF THE ACETABULUM AND OF AT LEAST PART OF THE PELVIS
IMPLANT POUR LA RECONSTRUCTION D'UNE ACÉTABULE ET DE PARTIE DU BASSIN

(30) Priorität: 16.02.2016 DE 102016202333
(43) Veröffentlichungstag der Anmeldung: 26.12.2018
(73) Patentinhaber: Waldemar Link GmbH & Co. KG, 22339 Hamburg (DE)
(72) Erfinder: LINK, Helmut D., 22397 Hamburg (DE)
(74) Vertreter: Hoffmann Eitle
(86) Internationale Anmeldenummer: PCT/EP2017/051901
(87) Internationale Veröffentlichungsnummer: WO 2017/140478

(56) Entgegenhaltungen:
- WO-A1-88/01491
- CN-A- 102 048 598
- JP-A- H0 523 363
- US-B1- 7 670 383

## Beschreibung

### GEBIET DER ERFINDUNG

Die vorliegende Erfindung betrifft ein Implantat zur Rekonstruktion bzw. zum Ersatz einer Hüftgelenkpfanne und zumindest eines Teils einer Beckenstruktur.

### STAND DER TECHNIK

Bei Patienten, die an einer starken Beschädigung oder gar dem Verlust eines Teils ihrer Beckenstruktur leiden, reichen die standardmäßig eingesetzten und etablierten künstlichen Hüftgelenkimplantate für eine Versorgung nicht aus. Durch den Verlust an Knochengewebe lassen sich derartige Endoprothesen nicht mehr ausreichend oder nur schwer verankern. Ein solcher Fall kann nach einer Revision oder gar wiederholter Revision einer Hüftendoprothese aufgrund von Abnutzung oder einer Entzündung auftreten. Andere Gründe für einen Verlust eines Teils der Beckenstruktur sind Unfälle oder Krebserkrankungen. Im Ergebnis macht eine derartige Situation eine patientenspezifische Lösung notwendig, die das verlorene oder beschädigte Knochengewebe ersetzen kann.

Ziel hierbei ist, den beschädigten oder fehlenden Teil der Beckenstruktur zu ersetzen oder zu rekonstruieren, damit das Becken wieder eine Gelenkpfanne für eines künstliches Hüftgelenk aufweist. Dies ist Voraussetzung dafür, dass die Bewegungsfähigkeit der Patientin oder des Patienten und somit Lebensqualität wieder zurückgewonnen werden.

Ein als "Tri-Flange Cup Implant" aus dem Stand der Technik bekannter Ansatz ist, den fehlenden Teil der Beckenstruktur naturgetreu zu rekonstruieren oder falls dies mangels ausreichender Informationen nicht möglich ist, diesen Teil durch das spiegelbildliche Gegenstück der Beckenstruktur möglichst gut anzunähern.

Allerdings hat eine derartig aufwändige Nachahmung der Beckenstruktur keinen ersichtlichen medizinischen Vorteil. Ein derartig angefertigtes aus Metall bestehendes Implantat ist um Größenordnungen schwerer und steifer als das native Original. Insbesondere der Ersatz des Darmbeins bedeutet den Ersatz eines durchaus erheblichen Knochenvolumens durch das Metall der Prothese. Das sich hieraus ergebende Implantat ist infolgedessen nicht nur schwer, sondern auch in Bezug auf Festigkeit erheblich überdimensioniert, da die Hauptaufgabe eines Darmbeinersatzes hauptsächlich darin liegt, die inneren Organe des Patienten abzustützen und so zum Beispiel zu verhindern, dass Hernien entstehen.

Insbesondere bei Ersatz eines größeren Abschnitts einer Beckenstruktur, wie zum Beispiel einer kompletten Seite eines Beckens einschließlich Darmbeins, Sitzbeins und Schambeins kann das Gewicht eines aus dem Stand der Technik bekannten Ersatzes zu Problemen bei der Verbindung mit bestehendem Knochengewebe führen. Insbesondere direkt nach dem Eingriff besteht die Gefahr, dass es bei den Verbindungsstellen für das Implantat zu Überlastungen und schlimmstenfalls zu weiteren Schäden am Gewebe des Patienten kommt.

Weiterhin bedeutet die Rekonstruktion der Knochengeometrie eine aufwändige Fertigung, die zwar eine patientenspezifische jedoch kostenintensive Möglichkeit für einen Ersatz der Beckenstruktur und des Hüftgelenks bereitstellt.

Ein Beispiel hierfür wird in der US 7,670,383 B1 offenbart, bei der eine Prothese zum Ersetzen eines Hüftknochens eine Acetabulum-Komponente beinhaltet, die eingerichtet ist, sich mit einem Femurimplantat und einem Schambein zu verbinden. Der für die Verbindung vorgesehene Schambeinteil an der frontalen Seite der Prothese ist lateral mit der Hüftgelenkpfanne verbunden und weist einen Klemmabschnitt auf, über den die Prothese an einem gegenüberliegenden gesunden Schambein befestigt werden kann.

### ZUSAMMENFASSUNG DER ERFINDUNG

Es war somit Aufgabe der vorliegenden Erfindung, ein Implantat bereitzustellen, welches vor allem die Hüftgelenkpfanne und zumindest einen Teil der Beckenstruktur ersetzt. Dabei wurde angestrebt, dass das Implantat eine geeignete Anbindung an bestehendes Knochengewebe ermöglicht und trotz Ersatz der fehlenden oder beschädigten Knochenstrukturen leichter ist als bekannte Lösungen. Weiterhin sollte das Implantat eine stabile Abstützung und Schonung der umliegenden Organe ermöglichen. Ein weiteres Ziel war zudem, trotz der hohen patientenspezifischen Anforderungen die Fertigungskosten und Konstruktionskosten eines auf den Patienten zugeschnittenen Implantats möglichst gering zu halten.

Um diesen Herausforderungen zu begegnen, stellt die vorliegende Erfindung ein Implantat zur Rekonstruktion einer Hüftgelenkpfanne und zumindest eines Teils einer Beckenstruktur bereit. Dabei weist das Implantat eine durch mindestens ein erstes Profilelement ausgebildete Rahmenstruktur für eine Übertragung von Gelenkkräften, einen Gelenkabschnitt, der zumindest einen Teil einer künstlichen Hüftgelenkpfanne ausbildet, mindestens zwei Anbindungsabschnitte für eine Anbindung des Implantats an Knochengewebe, wobei ein erster Anbindungsabschnitt für eine Anbindung an ein Kreuzbein oder Darmbein und ein zweiter Anbindungsabschnitt für eine Anbindung an ein Schambein vorgesehen ist, und mindestens ein Plattenelement zum Abstützen von inneren Organen auf, welches zumindest abschnittsweise durch die Rahmenstruktur umgeben wird.

Mit Hilfe der Rahmenstruktur und des mindestens einen Plattenelements werden die strukturellen Aufgaben des Implantats im Wesentlichen aufgeteilt. Dabei übernimmt die Rahmenstruktur vor allem die Übertragung der Gelenkkräfte zwischen dem Gelenkabschnitt und dem Knochengewebe bzw. dem Skelett des Patienten. Das mindestens eine Plattenelement dient dagegen vor allem zur Abstützung innerer Organe in Form einer zwischen der Rahmenstruktur aufgespannten und vorzugsweise raumgekrümmten Stütz- bzw. Trennwand.

Durch diesen Verbund ist es möglich, das Gewicht des Implantats erheblich zu reduzieren. Auch weist ein derart ausgeführtes Implantat eine geringere Steifigkeit als die oben genannten Tri-Flange-Implantate aus dem Stand der Technik auf. Infolgedessen ist die Steifigkeit des Implantats näher an der des zu ersetzenden Knochengewebes, was eine physiologischere Einleitung von Kräften über das Implantat in das anliegende bzw. angebundene Knochengewebe eines Patienten ermöglicht.

Dadurch, dass das Plattenelement zumindest abschnittsweise durch die Rahmenstruktur umgeben wird, beugt das die Rahmenstruktur ausbildende mindestens eine Profilelement einer Verletzung innerer Organe durch die umlaufende Kante des Plattenelements vor. Folglich sorgt der Verbund aus Plattenelement und Rahmenstruktur für eine zuverlässige Abstützung bei gleichzeitig gewebeschonendem Kontakt mit den inneren Organen und beugt so z. B. einem Auftreten von Hernien vor.

Damit nutzt das Implantat die Materialeigenschaften im Gegensatz zu anatomisch nachgebildeten Implantaten besser aus. Dies führt bezüglich der Festigkeit aber auch der Fertigung des Implantats zu einer funktionell orientierten Gestaltung. Insbesondere kann durch Querschnittsänderung und Materialstärke des Profils eine gezielte Verformung bzw. ein gezieltes elastisches Verhalten erreicht werden.

Je nach Ausmaß des Verlusts an Knochengewebe bzw. dessen Beschädigung kann das erfindungsgemäße Implantat neben der Hüftgelenkpfanne (Acetabulum) einen Teil des Schambeins (Os pubis) sowie einen Teil oder das gesamte Darmbein (Os ilium) ersetzen.

Die Anbindungsabschnitte ermöglichen dabei bei dem Implantat eine Anbindung an den Knochen, wobei die Anbindung als feste Verbindung und/oder als Abstützung vorgesehen sein kann.

Bei einer weiteren besonders bevorzugten Ausführungsform weist das Implantat eine zweite durch mindestens ein Profilelement ausgebildete Rahmenstruktur zum Ersatz von zumindest einem Teil eines Sitzbeins auf.

Diese bevorzugte Ausführungsform ermöglicht es, die Stützfunktion des Sitzbeins zu erhalten und damit ebenfalls zum Erhalt der Lebensqualität des Patienten beizutragen.

Gemäß einer weiteren Ausführungsform weist die zweite Rahmenstruktur ferner ein Plattenelement und/oder einen Anbindungsabschnitt auf.

Auch im Fall des Sitzbeins erfüllt das Plattenelement eine Stützfunktion und sorgt im Verbund mit dem mindestens einen Profilelement des Sitzbeins für eine gute Krafteinleitung in das Implantat. Falls nur ein Teil des Sitzbeins fehlt oder beschädigt ist, ermöglicht der Anbindungsabschnitt dieser Ausführungsform, den gesunden Teil des Sitzbeins zu erhalten.

Bei einer weiteren besonders bevorzugten Ausführungsform weist mindestens ein Anbindungsabschnitt zumindest abschnittsweise eine osseointegrierende Knochenkontaktfläche, insbesondere eine Trabekulärstruktur, und/oder eine osseoinduktive Knochenkontaktfläche auf.

Diese Ausführungsform ermöglicht es, das Implantat durch Einwachsen von Knochengewebe an dem Skelett des Patienten besonders zuverlässig zu verankern.

Dabei bietet eine osseointegrierende Knochenkontaktfläche dem Knochengewebe eine Struktur und/oder Oberfläche an, in die es Einwachsen bzw. sich mit dieser verbinden kann. Ein Beispiel für eine derartige Knochenkontaktfläche ist die Oberfläche einer Titanlegierung und/oder eine der nativen trabekulären Struktur des Knochengewebes angenäherte Oberfläche eines Anbindungsabschnitts.

Ist ein Anbindungsabschnitt osseoinduktiv, so bietet er dem anliegenden Knochengewebe nicht nur passiv eine biokompatible Oberfläche zum Ein- bzw. Anwachsen an, sondern begünstigt selbst aktiv durch entsprechende chemische Verbindungen oder Zytokine das Wachstum von Knochengewebe.

Bei einer weiteren Ausführungsform des Implantats der vorliegenden Erfindung weist der Gelenkabschnitt eine in etwa halbkugelförmige Aussparung auf, in die ein Gelenkeinsatz mit einer Gelenkfläche einsetzbar ist oder die eine Gelenkfläche ausbildet.

Eine derartige Ausführung des Gelenkabschnitts ermöglicht eine direkte oder indirekte Aufnahme des Hüftkopfes einer Hüftendoprothese. Bei der direkten Aufnahme bildet die halbkugelförmige Aussparung gleichzeitig eine Gelenkfläche aus. Bevorzugt erfolgt die Aufnahme des Gelenkkopfes jedoch indirekt, in dem in die halbkugelförmige Aussparung eine Gelenkpfanne mit einem Gelenkeinsatz oder auch nur ein Gelenkeinsatz eingesetzt ist. Hierdurch ist es möglich, diesen modular auszuführen, d.h. das ein und derselbe Gelenkabschnitt mehrere unterschiedliche Pfannen bzw. Einsätze aufnehmen kann, um die Gleitfläche an den zu verwendenden Hüftkopfersatz für den Patienten anzupassen. Außerdem fallen hierbei mögliche Verformungen im Falle eines Verschweißens des Gelenkabschnitts mit den Profilelementen und dem mindestens einen Plattenelement nicht so sehr ins Gewicht, da die für die Gelenkfläche notwendigen engen Toleranzen durch den Gelenkeinsatz eingehalten werden können.

Bevorzugt gibt die im Wesentlichen halbkugelförmige Aussparung in etwa die Außenkontur des Gelenkabschnitts vor. Mit anderen Worten weist der Gelenkabschnitt bevorzugt eine in etwa konstante Wandstärke auf, was insbesondere für ein Verschweißen der einzelnen Komponenten des erfindungsgemäßen Implantats von Vorteil ist. Noch bevorzugter ist die Wandstärke des Gelenkabschnitts unter Berücksichtigung des Durchmessers bzw. der Stärke der Profilelemente und/oder der Stärke des mindestens einen Plattenelements gewählt.

Bei einer besonders bevorzugten Ausführungsform der vorliegenden Erfindung ist mindestens eines der Profilelemente und sind vorzugsweise alle Profilelemente kantenfrei.

Durch eine derartige Gestaltung der Außenseite der Profilelemente bzw. ihres Querschnitts wird sichergestellt, dass im implantierten Zustand umliegendes Weichteilgewebe vor Verletzungen geschützt wird.

Mit anderen Worten weist bei dieser Ausführungsform mindestens eines der Profilelemente einen durchgehend abgerundeten bzw. stetig differenzierbaren Querschnitt auf. Folglich gibt es auf dem Umfang derartiger Profilelemente keine ebenen Flächen, die in einem Winkel aufeinander treffen und so eine möglicherweise gewebeschädigende Kante ausbilden können. Selbstverständlich ist es im Rahmen dieser Ausführungsform möglich, zwei ebene Flächen des Umfangs eines Profilelements in einem Winkel aufeinander stoßen zu lassen, solange der Stoß entsprechend abgerundet, also ein weicher Übergang zwischen den Flächen erzeugt wird. Gleiches gilt für die Stirnseiten eines Profilelements, wenn es auf die Stirnseite eines weiteren Profilelements trifft bzw. mit dieser verbunden ist.

Bei einer weiteren Ausführungsform der vorliegenden Erfindung ist das mindestens eine Profilelement der Rahmenstruktur zumindest abschnittsweise ein Hohlprofil.

Durch Ausbildung des mindestens einen Profilelements als Hohlprofil ist es möglich, die Wandstärke des Profilelements unter Berücksichtigung der Wandstärke des Gelenkabschnitts, des Plattenelements und/oder des Anbindungsabschnitts festzulegen und bevorzugt anzugleichen. Dadurch können Spannungsspitzen vermieden werden. Zudem hat dies insbesondere bei einer Verbindung der Profilelemente durch Schweißen miteinander und/oder mit anderen Komponenten des Implantats den Vorteil, dass hierdurch eine hohe Festigkeit der Schweißverbindung erreicht werden kann. Folglich ist zu bevorzugen, dass ein Profilelement zumindest in dem Bereich einer Schweißverbindung als Hohlprofil ausgeführt ist.

Ein weiterer Vorteil eines Hohlprofils ist eine damit verbundene Gewichtsreduktion. Dabei wird bevorzugt ein durchgehend als Hohlprofil ausgeführtes Profilelement und noch bevorzugter eine durchgehend als Hohlprofil ausgeführte Rahmenstruktur eingesetzt.

Weiterhin ist es von Vorteil, den mindesten einen Hohlraum der Rahmenstruktur hermetisch abgeschlossen auszuführen, um so zum Beispiel Entzündungen in diesem Bereich vorzubeugen. Zudem wird auch hierdurch der Entstehung möglicherweise scharfer Kanten vorgebeugt, die anderweitig durch den Zugang zu dem Hohlprofil entstehen könnten.

Bei einer weiteren bevorzugten Ausführungsform der vorliegenden Erfindung ist bei mindestens einem Anbindungsabschnitt mindestens ein Verbindungselement, insbesondere ein Durchgangsloch, für ein Befestigungselement vorgesehen.

Bei dieser Ausführungsform der Erfindung wird direkt während der Operation eine stabile Verbindung zwischen Implantat und Knochengewebe des Patienten erreicht. Dies hat insbesondere den Vorteil, dass ein Patient schnell wieder mobil ist, was den Heilungsprozess begünstigt und vorteilhaft für die Muskulatur und den Bänderapparat ist.

Wenn der jeweilige Anbindungsabschnitt zudem einen osseointegrierenden und/oder osseoinduktiven Teil aufweist, sorgt diese stabile Verbindung zudem für die für ein zuverlässiges Einwachsen des Knochens notwendige Primärstabilität.

Vorzugsweise handelt es sich bei dem Verbindungselement um ein Durchgangsloch, das durch einen Anbindungsabschnitt für die Aufnahme eines Befestigungselements ausgebildet ist. Als Befestigungselement können beispielsweise Schrauben, Klammern oder Drähte verwendet werden.

Bei einer weiteren besonders bevorzugten Ausführungsform grenzt mindestens ein Plattenelement abschnittsweise an einen Anbindungsabschnitt und/oder einen Gelenkabschnitt an.

Hierdurch wird sichergestellt, dass ein Plattenelement größtenteils und vorzugsweise durchgehend an seinem Umfang mit einem Profilelement, Anbindungsabschnitt und/oder Gelenkabschnitt umgeben ist und so an das Implantat angrenzendes Gewebe vor Beschädigung geschützt wird.

Bei einer besonders bevorzugten Ausführungsform des Implantats sind für die Übertragung der Gelenkkräfte von dem Gelenkabschnitt im Wesentlichen zwei gekrümmte Profilelemente vorgesehen, die jeweils auf einer ersten Seite mit einem Anbindungselement und auf der dazu gegenüberliegenden zweiten Seite mit dem Gelenkabschnitt verbunden sind, wobei die erste und zweite Seite von jedem der Profilelemente vorzugsweise in entgegengesetzte Richtungen zeigen.

Durch die Krümmung der Profilelemente wird dabei einerseits eine genauere Rekonstruktion der Beckenstruktur erreicht und andererseits die Einleitung der Gelenkkräfte gegenüber geraden Profilen abgefedert.

Darunter, dass die erste Seite und die zweite Seite eines Profilelements in entgegengesetzte Richtungen zeigen ist zu verstehen, dass die aus den Stirnseiten des Profilelements austretende Längsachse des Profils bzw. die darauf liegenden von dem Profilelement weg zeigenden Vektoren in einem Winkel zueinander stehen. Durch die Biegung des jeweiligen Profilelements beträgt dieser somit auf einer Seite weniger als 180°, jedoch sollte er 90° und bevorzugt 135° nicht unterschreiten.

Zudem stellt die vorliegende Erfindung ein Verfahren zur Herstellung eines patientenspezifischen Implantats bereit, das für die Rekonstruktion eines Gelenks und zumindest eines Teils einer angrenzenden Knochenstruktur geeignet ist. Hierfür umfasst das Verfahren die folgenden Schritte. Das Implantat wird mit mindestens einem Anbindungsabschnitt für eine Anbindung des Implantats an Knochengewebe, einer Rahmenstruktur, die mindestens ein Profilelement aufweist, mindestens einem Plattenelement, das zumindest abschnittsweise durch die Rahmenstruktur umgeben wird, und mindestens einem Gelenkabschnitt, der zumindest einen Teil eines künstlichen Gelenks ausbildet, gestaltet. Für das so gestaltete Implantat werden Formdaten erstellt, auf deren Grundlage das Implantat anschließend hergestellt wird.

Der strukturelle Verbund aus dem mindestens einen Plattenelement und dem mindestens einen Profilelement der Rahmenstruktur ist eine patientenspezifische Lösung zum Ersatz einer Knochenstruktur, die schnell und kostengünstig hergestellt werden kann. Dabei kann gegebenenfalls die Form der Anbindungsabschnitte an das Knochengewebe, an dem sie verankert werden, angepasst werden.

Die einzelnen Elemente und Abschnitte bieten dabei einen Baukasten struktureller Elemente, mit denen fehlendes Knochengewebe auf einfach Weise rekonstruiert werden kann. Danach können die Elemente und Anbindungsabschnitte entweder direkt als ein Körper hergestellt werden oder vorgefertigt und anschließend miteinander verbunden werden.

Bei einer besonders bevorzugten Ausführungsform der vorliegenden Erfindung wird mindestens ein Profilelement und/oder Plattenelement dreidimensional gebogen.

Hierdurch kann mithilfe des Herstellungsverfahrens das herzustellende Implantat besonders gut an die Anatomie eines Patienten angepasst werden. Mit anderen Worten kann durch das dreidimensionale Biegen jegliche beliebige Außenkontur eines Teils der zu rekonstruierenden Beckenstruktur funktionell und kostengünstig nachgebildet und/oder angepasst werden. Hierdurch wird eine optimale Abstützung der inneren Organe im Bereich des Beckenbodens sowie eine funktionell orientierte Weiterleitung von Kräften, wie zum Beispiel der Gelenkkräfte, erreicht. Insbesondere kann durch das dreidimensionale Biegen eine abgefederte Krafteinleitung in das Skelett umgesetzt werden, da sich das Profilelement zwischen dem Gelenkabschnitt und einem Anbindungsabschnitt befindet.

Unter dreidimensionalem bzw. räumlichem Biegen ist dabei das Hervorrufen einer Krümmung zu verstehen, die nicht in einer einzigen planen Ebene liegt, sondern für ihre Beschreibung drei Raumrichtungen benötigt.

Bei einer besonders bevorzugten Ausführungsform der vorliegenden Erfindung wird bzw. werden die Rahmenstruktur, der Gelenkabschnitt und/oder das mindestens eine Plattenelement über eine Schweißverbindung verbunden.

Dadurch, dass die Profilelemente der Rahmenstruktur, der Gelenkabschnitt und/oder das mindestens eine Plattenelement untereinander und/oder miteinander über Schweißverbindungen fest verbunden werden, wird eine durchgehend gewebeschonende Abstützung der inneren Organe erreicht, da bei Schweißverbindungen im Gegensatz zu Befestigungselementen keine Vorsprünge, wie beispielsweise Schraubenköpfe, vorhanden sind. Zudem handelt es sich bei Schweißverbindungen um eine nicht lösbare Verbindung, die eine lange Lebenszeit des Implantats ermöglicht. Auch handelt es sich bei Schweißverbindungen um eine höchst anpassungsfähige Verbindungstechnik, die bei dem Implantat eine kostengünstige Herstellung ermöglicht.

Bei einer weiteren besonders bevorzugten Ausführungsform der vorliegenden Erfindung wird das Implantat mittels eines additiven Herstellungsverfahrens hergestellt.

Die Verwendung eines additiven Herstellungsverfahrens hat vor allem den Vorteil, dass es mit relativ geringem Aufwand auch eine patientenspezifische komplexe Implantatgeometrie mit relativ geringem Kostenaufwand herstellen kann. Insbesondere ist es mit einem solchen Herstellungsverfahren besonders einfach, die oben erwähnten, möglicherweise im Implantat vorhandenen, in sich hermetisch abgeschlossene Hohlräume herzustellen. Ein mit einem solchen Verfahren hergestelltes Implantat ist vorzugsweise einstückig.

Bei einer weiteren Ausführungsform der vorliegenden Erfindung ist mindestens ein Plattenelement, der Gelenkabschnitt und/oder die Rahmenstruktur zumindest abschnittsweise oberflächenbehandelt.

Unter Oberflächenbehandlung ist bzw. sind im Rahmen der vorliegenden Erfindung eine gezielte Gestaltung der Oberfläche des Implantatmaterials und/oder eine Beschichtung desselben zu verstehen. Auf diese Weise kann zum Beispiel eine osseointegrierende oder osseoinduktive Eigenschaft eines Anbindungsabschnitts erreicht werden. Auf ähnliche Weise kann dafür gesorgt werden, dass Bindegewebe an dem Implantat anhaftet und so die inneren Organe noch besser von dem Implantat abgestützt werden. Weiterhin ist es durch die Oberflächenbehandlung möglich, allergischen Reaktionen gegen das Implantatmaterial vorzubeugen.

### KURZE BESCHREIBUNG DER FIGUREN

In den begleitenden Figuren, auf die im Folgenden im Rahmen der ausführlichen Beschreibung derzeit bevorzugter Ausführungsformen Bezug genommen wird, werden Elemente mit gleicher Funktion und/oder Gestaltung mit gleichen Bezugszeichen gekennzeichnet. Dabei zeigt
Figur 1 ein erfindungsgemäßes in eine Beckenstruktur integriertes Implantat schräg von vorne, das einen Teil einer Beckenstruktur ersetzt,
Figur 2 eine seitliche Ansicht einer erfindungsgemäßen Beckenstruktur aus Figur 1 von der linken Seite eines Patienten aus,
Figur 3 eine seitliche Ansicht einer erfindungsgemäßen Beckenstruktur von medial; und
Figur 4 eine repräsentative native Beckenstruktur von vorne.

### AUSFÜHRLICHE BESCHREIBUNG BEVORZUGTER AUSFÜHRUNGSFORMEN

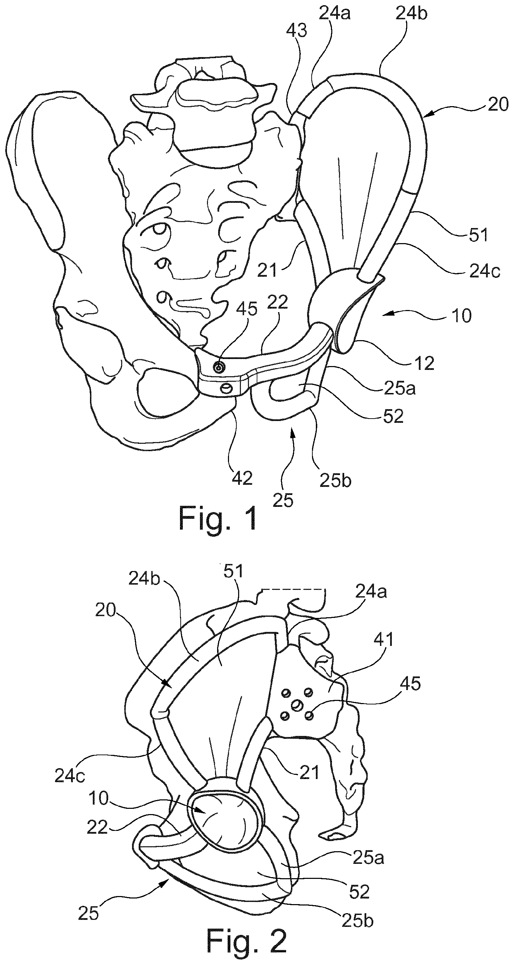

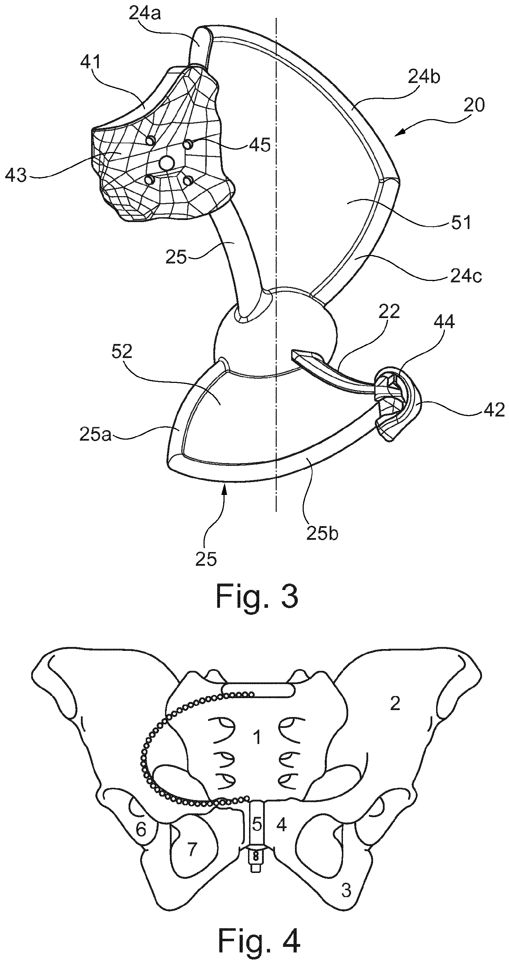

Figur 1 zeigt ein erfindungsgemäßes Implantat für einen teilweisen Ersatz einer Beckenstruktur einschließlich der Hüftgelenkpfanne im implantierten Zustand. Genauer gesagt werden bei der in den Figuren 1 bis 3 gezeigten beispielhaften Ausführungsform des Implantats das Darmbein 2, das Sitzbein 3, das Schambein 4, die Schambeinfuge 5, und insbesondere die Hüftgelenkpfanne 6 ersetzt (siehe Figur 4).

Das Implantat ist an einer Stelle mit einem an dem Ersatz des Darmbeins 2 vorgesehenen Anbindungsabschnitt 41 mit dem Kreuzbein 1 des Patienten verbunden. Allerdings kann je nach Ausmaß des beschädigten oder fehlenden Knochengewebes die Anbindung bei dem Anbindungsabschnitt 41 auch an einer Stelle am Darmbein erfolgen.

Ferner ist das Implantat in Figur 1 an einer zweiten Stelle über einen weiteren Anbindungsabschnitt 42 mit dem Schambein 4 der Seite der Beckenstruktur verbunden, welche der zu ersetzenden Seite des Beckens gegenüberlegt. Genauso kann die Anbindung des Anbindungsabschnitts 42 für das Schambein an dem Teil des Schambeins angebunden sein, der sich auf der Seite des zu ersetzenden Acetabulums befindet. Hierdurch wird die Schambeinfuge erhalten und ermöglicht eine nachgiebige und dämpfende Aufnahme von Belastungen und insbesondere von Schwingungen.

Je nach Ausmaß des zu ersetzenden Gewebes kann es auch sinnvoll sein, dass der Anbindungsabschnitt 42 nicht mit dem Schambein verbunden ist, sondern stattdessen mit dem auf der gegenüberliegenden Seite des zu ersetzenden Beckens befindlichen Darmbein 2 oder Kreuzbein 1. In einem solchen Fall werden dementsprechend beide Hüftgelenkpfannen 6 ersetzt.

Wie in Figur 1 zu erkennen ist, wurde die Hüftgelenkpfanne 6 bzw. das Acetabulum durch einen Gelenkabschnitt 10 ersetzt. Dieser weist eine in etwa halbkugelförmige Aussparung 12 auf, die, wie oben beschrieben, für eine Aufnahme eines Hüftgelenkkopfes vorgesehen ist. Im veranschaulichten Fall weist der Gelenkabschnitt 10 zudem auch auf der gegenüberliegenden bzw. Außenseite in etwa die Form einer Halbkugel auf, sodass die Wandstärke des Gelenkabschnitts 10 in etwa konstant ist.

Die Verbindung des Gelenkabschnitts 10 mit dem Anbindungsabschnitt 41 des Darmbeinersatzes erfolgt über das Profilelement 21 und die Verbindung des Gelenkabschnitts 10 mit dem Anbindungsabschnitt 42, der im veranschaulichten Fall mit dem rechten Schambein des Patienten verbunden ist, erfolgt über das Profilelement 22.

Wie in der Figur 1 veranschaulicht ist, weist der Anbindungsabschnitt 42 ein Verbindungselement 45 auf, das bei der vorliegenden beispielhaften Ausführungsform als Durchgangsloch ausgeführt ist. Die in dem veranschaulichten Anbindungsabschnitt 42 vorgesehenen zwei Durchgangslöcher 45 dienen zur Befestigung des Implantats über Befestigungselemente, wie zum Beispiel Knochenschrauben, Stifte, Klammern oder Drähte. Allgemein werden mindestens zwei Verbindungselemente bevorzugt - einerseits, um eine feste Anbindung zu erreichen, und andererseits, um intraoperativ, falls notwendig, eine Auswahlmöglichkeit für eine optimale Befestigung des Implantats an Knochengewebe zur Verfügung zu haben. Hierdurch wird bereits während der Operation eine stabile Befestigung des Implantats an dem Skelett des Patienten erreicht.

Auf der anderen Seite des Gelenkabschnitts weist der Anbindungsabschnitt 41 ebenfalls Verbindungselemente 45 in Form von Durchgangslöchern auf (Figur 2), mit denen während des operativen Eingriffs eine feste Verbindung des Implantats mit dem Kreuzbein des Patienten erreicht wird (Primärstabilität).

Wie in Figur 3 zu erkennen ist, können der Anbindungsabschnitt des Implantats für das Darmbein 41 und/oder der Anbindungsabschnitt 42 für das Schambein eine an die jeweilige Knochengeometrie angepasste Oberfläche 43 bzw. 44 aufweisen. Dabei sind die mit dem Knochengewebe in Kontakt stehenden Flächen der Anbindungsabschnitte 41 und 42 bevorzugt osseointegrierend und/oder osseoinduktiv ausgeführt. Ist dies der Fall, wird das Implantat möglicherweise zusätzlich zu den Verbindungselementen 45 durch Anlagerung bzw. Einwachsen von Knochengewebe befestigt, wodurch eine sogenannte Sekundärstabilität erreicht wird. Dies gilt auch für den Fall, bei dem die dem Knochengewebe zugewandte Fläche des Anbindungsabschnitts 41 und/oder 42 nicht patientenspezifisch an die Oberfläche des anliegenden Knochengewebes angepasst ist, sondern stattdessen die Oberfläche des Knochengewebes während eines operativen Eingriffs bei dem Patienten an den jeweiligen Anbindungsabschnitt angepasst wird.

Abweichend von der dargestellten Ausführungsform kann mindestens ein Anbindungsabschnitt auch dazu vorgesehen sein, eine Anbindung an das Knochengewebe lediglich durch ein Abstützen zu erreichen.

Neben den Profilelementen 21 und 22 weist das in den Figuren 1 bis 3 dargestellte Implantat bei dem Darmbeinersatz zudem weitere Profilelemente 24a, 24b, 24c auf, die, wie oben beschrieben, vorzugsweise über Schweißverbindungen verbunden sind.

Es ist auch möglich, einige oder sämtliche dieser Profilelemente 24a, 24b, 24c durch ein einziges Profilelement zu ersetzen, das entsprechend gebogen bzw. verformt wird. Wie insbesondere in den Figuren 1 und 2 zu erkennen, sind die Profilelemente 24a, 24b, 24c an ihren Verbindungsstellen abgerundet, um im implantierten Zustand gegen eine Verletzung umliegenden Gewebes vorzubeugen. Die Profilelemente 24a, 24b, 24c bilden im Wesentlichen die kraniale Außenkontur des nativen Darmbeins nach.

Weiterhin weist der Darmbeinersatz ein Plattenelement 51 auf, das insbesondere für eine Abstützung der inneren Organe vorgesehen ist. Um das Gewebe des Patienten vor der umlaufenden Kante des Plattenelements 51 zu schützen, ist dieses einerseits durch die Rahmenstruktur 21, 24a, 24b, 24c, wie auch durch den Gelenkabschnitt 10 und den Anbindungsabschnitt 41 vollständig umschlossen.

Dementsprechend weist das mindestens eine Profilelement der Rahmenstruktur einen Querschnitt auf, der zumindest in der Abmessung senkrecht zum Plattenelement größer bzw. dicker ist. Vorzugsweise beträgt die Dicke eines Profilelements entlang des mindestens einen Plattenelements mindestens das zweifache, vorzugsweise mindestens das fünffache und am bevorzugtesten mindestens das zehnfache der Dicke dieses Plattenelements.

Wie insbesondere in Figur 2 zu erkennen, ist der Anbindungsabschnitt 41 ebenfalls als Plattenelement ausgeführt. Allerdings ist es bei einem derartig plattenförmig ausgeführten Anbindungsabschnitt nicht notwendig, diesen ebenfalls durch Profilelemente zu umgeben, da es sich bei den Kanten des Anbindungsabschnitts 41 nicht um freistehende Kanten handelt. Mit anderen Worten liegen die Kanten des Anbindungsabschnitts 41 direkt am Knochengewebe an, sodass hierdurch einer Gefährdung innerer Organe vorgebeugt wird. Die Kanten des Anbindungsabschnitts 41 sind vorzugsweise abgerundet.

Bei dem in den Figuren 1 bis 3 veranschaulichten Implantat ist zudem ein vollständiger Ersatz 25 des nativen Sitzbeins 3 vorgesehen. Dabei weist der Sitzbeinersatz 25 die beiden Profilelemente 25a, 25b auf sowie ein weiteres Plattenelement 52. Durch diese Ergänzung stellt die beispielhafte Ausführungsform des Implantats eine umfassende Aufrechterhaltung der durch die native Beckenstruktur bereitgestellten Stützfunktion bereit (vgl. Figur 4), was von erheblichem Vorteil ist.

Abweichend von der dargestellten Ausführungsform des Implantats kann das Plattenelement 52 bei einem Ersatz des Sitzbeins 3 auch weggelassen oder umgestaltet werden, um so insbesondere ein Hindurchführen oder Befestigen von Strukturen zu ermöglichen, die sich bei der nativen Beckenstruktur in diesem Bereich befinden.

Falls der durch das Profilelement 25b ersetzte Teil des nativen Sitzbeins 3 und Schambeins 4 erhalten werden soll (vgl. Figur 4), kann bei dem als Sitzbeinersatz vorgesehenen Teil des Implantats ein weiterer nicht gezeigter Anbindungsabschnitt vorgesehen sein.

Falls ein derartiger Knochenersatz notwendig ist oder um eine möglichst stabile Anbindung an das Knochengewebe zu erreichen, kann es vorteilhaft sein, das gesamte Sitzbein und Schambein einer Seite der Beckenstruktur zu ersetzen, wie in Figur 1 gezeigt.

Weiterhin ist anzumerken, dass das erfindungsgemäße Beckenimplantat, wie oben beschrieben, die native Beckenstruktur bzw. einen Teil davon nicht nur funktionell ersetzen kann, sondern zudem insbesondere im Rahmen einer additiven und/oder adaptiven Fertigung kostengünstig herstellbar ist. Nach der rechnergesteuerten Planung eines erfindungsgemäßen patientenspezifischen Implantats kann die darauffolgende Fertigung nahezu vollständig automatisch ablaufen. Auf diese Weise ist es möglich, das Implantat schnell, dem jeweiligen Patienten angepasst und kostengünstig zur Verfügung zu stellen.

Je nach angewandter Fertigungstechnik des Implantats können die Plattenelemente, Profilelemente und/oder Anbindungsabschnitte zumindest teilweise vor dem Zusammenbau des Implantats vorgeformt werden, um so die Montage der einzelnen Komponenten insbesondere bei Anwendung von Schweißtechnik zu erleichtern.

Besonders bevorzugt wird allerdings ein additives Verfahren zur Herstellung des Implantats eingesetzt.

Insbesondere sind hierfür das Elektronenstrahlschmelzen (EBM - Electron Beam Melting) und das selektive Laserschmelzen (SLM - Selective Laser Melting) geeignet. Bei beiden Verfahren wird ein zu verarbeitendes Metallpulver in einer dünnen Schicht auf einer Grundplatte aufgebracht. Dann wird der pulverförmige Werkstoff mittels eines Elektronenstrahls oder Laserstrahls lokal aufgeschmolzen oder belichtet und bildet nach Erstarrung eine feste Materialschicht aus. Anschließend wird die Grundplatte um den Betrag einer Schichtdicke abgesenkt und erneut Pulver aufgetragen. Dieser Zyklus wird solange wiederholt, bis alle Schichten umgeschmolzen sind.

Weiterhin ist es möglich, mit Hilfe eines additiven Verfahrens eine Gussform herzustellen, mit der das Implantat anschließend urgeformt wird. Genauso kann das Implantat über ein additives Verfahren als Grünling erzeugt werden, der anschließend gesintert wird.

Der Einsatz eines additiven Verfahrens ist im Rahmen des Beckenersatzes vor allem deswegen von Vorteil, da durch die Gestaltungsfreiheit bei der Erstellung der Struktur Spannungen gleichmäßig verteilt werden können und so die Materialbelastung auf einem niedrigen Niveau gehalten werden kann. Folglich kann die Werkstofffestigkeit verringert werden. Dies trifft vor allem auf das EBM- und SLM-Verfahren zu, da bei beiden Verfahren keine Gusskerne entfernt werden müssen, kein Materialfließverhalten bei der Gestaltung des Ersatzes beachtet werden muss und zudem beispielsweise in sich abgeschlossene Hohlräume erzeugt werden können. Auch wird bei diesen Verfahren direkt das Endprodukt hergestellt anstatt eines Zwischenprodukts mittels dessen das Endprodukt erst noch gefertigt wird.

Weiterhin kann, wie oben bereits beschrieben, die Oberfläche des mindestens einen Plattenelements, der Rahmenstruktur und/oder des Gelenkabschnitts zumindest abschnittsweise gezielt behandelt werden, um so eine Verbindung des umliegenden Weichteilgewebes bzw. Bindegewebes mit dem Implantat zu ermöglichen und dadurch die Stützfunktion, insbesondere des Darmbeinersatzes, weiter zu verbessern.

Bei vorhandenem Plattenelement 52 im Bereich des Sitzbeinersatzes kann es vorteilhaft sein, dass dieses die durch die Profilelemente 25a, 25b, 22 und den Gelenkabschnitt 10 gebildete Umrandung nur abschnittsweise ausfüllt, um so eine Anbindung von Muskulatur, Sehnen und/oder Bändern zu ermöglichen.

### BEZUGSZEICHEN

- 1: Kreuzbein (Os sacrum)
- 2: Darmbein (Os ilium)
- 3: Sitzbein (Os ischii)
- 4: Schambein (Os pubis)
- 5: Schambeinfuge (Symphysis pubis)
- 6: Hüftgelenkpfanne (Acetabulum)
- 7: Foramen obturatum
- 8: Steißbein (Os coccygis)
- 10: Gelenkabschnitt
- 12: Gelenkaussparung
- 20: erste Rahmenstruktur
- 21: Profilelement zwischen Gelenk- und Kreuzbein-/ Darmbeinanbindungsabschnitt
- 22: Profilelement zwischen Gelenk- und Schambeinanbindungsabschnitt
- 24a, 24b, 24c: Profile für das Darmbein
- 25: zweite Rahmenstruktur
- 25a, 25b: Sitzbeinprofile
- 41: Anbindungsabschnitt zum Kreuzbein/Darmbein
- 42: Anbindungsabschnitt zum Schambein
- 43, 44: Knochenkontaktfläche
- 45: Verbindungselement für Befestigungselement
- 51: Plattenelement für den Ersatz des Darmbeins
- 52: Plattenelement zum mindestens teilweisen Verschließen des Sitzbeins

## Patentansprüche

1. Implantat zur Rekonstruktion einer Hüftgelenkpfanne und zumindest eines Teils einer Beckenstruktur, wobei das Implantat aufweist:
eine durch mindestens ein erstes Profilelement (21, 22, 24) ausgebildete Rahmenstruktur (20) für eine Übertragung von Gelenkkräften,
einen Gelenkabschnitt (10), der zumindest einen Teil einer künstlichen Hüftgelenkpfanne (12) ausbildet,
mindestens zwei Anbindungsabschnitte (41, 42) für eine Anbindung des Implantats an Knochengewebe, wobei ein erster Anbindungsabschnitt (41) für eine Anbindung an ein Kreuzbein oder Darmbein und ein zweiter Anbindungsabschnitt für eine Anbindung an ein Schambein (42) vorgesehen ist, und
mindestens ein Plattenelement (51) zum Abstützen von inneren Organen, dessen umlaufende Kante zumindest abschnittsweise durch die Rahmenstruktur (20) umgeben ist.

2. Implantat nach Anspruch 1, wobei das Implantat eine zweite durch mindestens ein Profilelement (26, 27) ausgebildete Rahmenstruktur (25) zum Ersatz von zumindest einem Teil eines Sitzbeins aufweist.

3. Implantat nach Anspruch 2, wobei die zweite Rahmenstruktur ferner ein Plattenelement (52) und/oder einen Anbindungsabschnitt aufweist.

4. Implantat nach einem der vorstehenden Ansprüche, bei dem mindestens ein Anbindungsabschnitt (41, 42) zumindest abschnittsweise eine osseointegrierende Knochenkontaktfläche (43, 44), insbesondere eine Trabekulärstruktur, und/oder eine osseoinduktive Knochenkontaktfläche aufweist.

5. Implantat nach einem der vorstehenden Ansprüche, bei dem der Gelenkabschnitt (10) eine in etwa halbkugelförmige Aussparung (12) aufweist, in die ein Gelenkeinsatz mit einer Gelenkfläche einsetzbar ist oder die eine Gelenkfläche ausbildet.

6. Implantat nach einem der vorstehenden Ansprüche, bei dem mindestens ein Profilelement, vorzugsweise alle Profilelemente, kantenfrei ist bzw. sind.

7. Implantat nach einem der vorstehenden Ansprüche, bei dem mindestens ein Profilelement (21, 22, 24, 26, 27) der Rahmenstruktur (20, 25) zumindest abschnittsweise als Hohlprofil ausgebildet ist.

8. Implantat nach einem der vorstehenden Ansprüche, bei dem bei mindestens einem Anbindungsabschnitt mindestens ein Verbindungselement (45), insbesondere ein Durchgangsloch, für ein Befestigungselement vorgesehen ist.

9. Implantat nach einem der vorstehenden Ansprüche, bei dem mindestens ein Plattenelement abschnittsweise an einen Anbindungsabschnitt und/oder Gelenkabschnitt angrenzt.

10. Implantat nach einem der vorstehenden Ansprüche, bei dem im Wesentlichen zwei gekrümmte Profilelemente (21, 22) für die Übertragung der Gelenkkräfte von dem Gelenkabschnitt (10) vorgesehen sind, wobei die Profilelemente (21, 22) jeweils an einem Ende mit einem Anbindungselement (41, 42) und an dem diesem gegenüberliegenden zweiten Ende mit dem Gelenkabschnitt (10) verbunden sind, wobei das erste und zweite Ende von jedem der Profilelemente vorzugsweise in entgegengesetzte Richtungen zeigen.

11. Verfahren zur Herstellung eines patientenspezifischen Beckenimplantats, das für die Rekonstruktion eines Gelenks und zumindest eines Teils einer angrenzenden Knochenstruktur geeignet ist, wobei das Verfahren die Schritte umfasst:
- Gestalten des Implantats mit mindestens einem Anbindungsabschnitt für eine Anbindung des Implantats an Knochengewebe, mindestens einer Rahmenstruktur (20), die mindestens ein Profilelement (21, 22, 24) aufweist, mindestens einem Plattenelement (51), dessen umlaufende Kante zumindest abschnittsweise durch die Rahmenstruktur (20) umgeben wird, und mindestens einem Gelenkabschnitt (10), der zumindest einen Teil eines künstlichen Gelenks ausbildet,
- Erstellen von Formdaten des so gestalteten Implantats, und
- Herstellen des Implantats auf Grundlage der Formdaten.

12. Verfahren nach Anspruch 11, bei dem mindestens ein Profilelement und/oder Plattenelement dreidimensional gebogen wird.

13. Verfahren nach Anspruch 11 oder 12, bei dem die Rahmenstruktur (20, 25), der Gelenkabschnitt (10) und/oder das mindestens eine Plattenelement (51, 52) über eine Schweißverbindung verbunden werden.

14. Verfahren nach einem der Ansprüche 11 bis 13, bei dem zumindest ein Abschnitt des Implantats mittels eines additiven Herstellungsverfahrens hergestellt wird.

15. Verfahren nach einem der Ansprüche 11 bis 14, bei dem ein Plattenelement, der Gelenkabschnitt und/oder die Rahmenstruktur zumindest abschnittsweise oberflächenbehandelt wird bzw. werden.

## Claims

1. Implant for reconstructing an acetabulum and at least part of a pelvic structure, wherein the implant has:
a frame structure (20), which is formed by at least one first profile element (21, 22, 24) and serves to transmit joint forces,
a joint portion (10), which forms at least part of an artificial acetabulum (12),
at least two attachment portions (41, 42) for attaching the implant to bone tissue, wherein a first attachment portion (41) is provided for attachment to a sacrum or ilium and a second attachment portion is provided for attachment to a pubis (42), and
at least one plate element (51) for supporting internal organs, the circumferential edge of which plate element (51) is surrounded at least in regions by the frame structure (20).

2. Implant according to Claim 1, wherein the implant has a second frame structure (25), which is formed by at least one profile element (26, 27) and serves to replace at least part of an ischium.

3. Implant according to Claim 2, wherein the second frame structure moreover has a plate element (52) and/or an attachment portion.

4. Implant according to one of the preceding claims, in which at least one attachment portion (41, 42) has, at least in regions, an osseointegrative bone contact surface (43, 44), in particular a trabecular structure, and/or an osteoinductive bone contact surface.

5. Implant according to one of the preceding claims, in which the joint portion (10) has an approximately hemispherical recess (12), into which a joint insert with an articular surface can be inserted or which forms an articular surface.

6. Implant according to one of the preceding claims, in which at least one profile element, preferably all the profile elements, is/are free from edges.

7. Implant according to one of the preceding claims, in which at least one profile element (21, 22, 24, 26, 27) of the frame structure (20, 25) is configured at least in regions as a hollow profile.

8. Implant according to one of the preceding claims, in which at least one connecting element (45), in particular a through-hole, for a fastening element is provided in at least one attachment portion.

9. Implant according to one of the preceding claims, in which at least one plate element in some regions adjoins an attachment portion and/or joint portion.

10. Implant according to one of the preceding claims, in which substantially two curved profile elements (21, 22) are provided for transmitting the joint forces from the joint portion (10), wherein the profile elements (21, 22) are each connected at one end to an attachment element (41, 42) and, at the second end opposite this one, are connected to the joint portion (10), wherein the first and second end of each of the profile elements preferably point in opposite directions.

11. Method for manufacturing a patient-specific pelvic implant that is suitable for reconstructing a joint and at least part of an adjacent bone structure, wherein the method comprises the steps of:
- designing the implant with at least one attachment portion for attaching the implant to bone tissue, with at least one frame structure (20), which has at least one profile element (21, 22, 24), with at least one plate element (51), of which the circumferential edge is surrounded at least in regions by the frame structure (20), and with at least one joint portion (10), which forms at least part of an artificial joint,
- preparing shape data of the implant thus designed; and
- manufacturing the implant on the basis of the shape data.

12. Method according to Claim 11, in which at least one profile element and/or plate element is bent three-dimensionally.

13. Method according to Claim 11 or 12, in which the frame structure (20, 25), the joint portion (10) and/or the at least one plate element (51, 52) are connected by a welded joint.

14. Method according to one of Claims 11 to 13, in which at least one portion of the implant is manufactured by means of an additive manufacturing process.

15. Method according to one of Claims 11 to 14, in which a plate element, the joint portion and/or the frame structure is/are surface-treated at least in some regions.

## Revendications

1. Implant pour la reconstruction d'une acétabule de hanche et d'au moins une partie d'une structure du bassin, l'implant présentant :
une structure de cadre (20) réalisée par au moins un premier élément profilé (21, 22, 24) pour un transfert de forces d'articulation,
une portion d'articulation (10) qui constitue au moins une partie d'une acétabule de hanche artificielle (12),
au moins deux portions de liaison (41, 42) pour une liaison de l'implant au tissu osseux, une première portion de liaison (41) étant prévue pour une liaison à un sacrum ou à un os iliaque et une deuxième portion de liaison étant prévue pour une liaison à un pubis (42), et
au moins un élément de plaque (51) pour le support d'organes internes, dont l'arête périphérique est entourée au moins en partie par la structure de cadre (20).

2. Implant selon la revendication 1, l'implant présentant une deuxième structure de cadre (25) réalisée par au moins un élément profilé (26, 27) pour remplacer au moins une partie d'un ischion.

3. Implant selon la revendication 2, dans lequel la deuxième structure de cadre présente en outre un élément de plaque (52) et/ou une portion de liaison.

4. Implant selon l'une quelconque des revendications précédentes, dans lequel au moins une portion de liaison (41, 42) présente au moins en partie une surface de contact de l'os osséointégrative (43, 44), en particulier une structure trabéculaire, et/ou une structure de contact de l'os osséoinductive.

5. Implant selon l'une quelconque des revendications précédentes, dans lequel la portion d'articulation (10) présente un évidement (12) de forme approximativement hémisphérique dans lequel peut être inséré un insert d'articulation avec une surface d'articulation ou qui constitue une surface d'articulation.

6. Implant selon l'une quelconque des revendications précédentes, dans lequel au moins un élément profilé, de préférence tous les éléments profilés, sont exempts d'arêtes.

7. Implant selon l'une quelconque des revendications précédentes, dans lequel au moins un élément profilé (21, 22, 24, 26, 27) de la structure de cadre (20, 25) est réalisé au moins en partie sous forme de profilé creux.

8. Implant selon l'une quelconque des revendications précédentes, dans lequel, dans le cas d'au moins une portion de liaison, au moins un élément de connexion (45), en particulier un trou traversant, est prévu pour un élément de fixation.

9. Implant selon l'une quelconque des revendications précédentes, dans lequel au moins un élément de plaque est en partie adjacent à une portion de liaison et/ou une portion d'articulation.

10. Implant selon l'une quelconque des revendications précédentes, dans lequel essentiellement deux éléments profilés courbes (21, 22) sont prévus pour le transfert des forces d'articulation depuis la portion d'articulation (10), les éléments profilés (21, 22) étant à chaque fois connectés par une extrémité à un élément de liaison (41, 42) et par la deuxième extrémité opposée à celle-ci à la portion d'articulation (10), la première et la deuxième extrémité de chacun des éléments profilés étant de préférence tournées dans des directions opposées.

11. Procédé de fabrication d'un implant de bassin spécifique à un patient, qui est approprié pour la reconstruction d'une articulation et d'au moins une partie d'une structure osseuse adjacente, le procédé comprenant les étapes suivantes :
- configuration de l'implant avec au moins une portion de liaison pour une liaison de l'implant à des tissus osseux, au moins une structure de cadre (20) qui présente au moins un élément profilé (21, 22, 24), au moins un élément de plaque (51) dont l'arête périphérique est entourée au moins en partie par la structure de cadre (20), et au moins une portion d'articulation (10) qui constitue au moins une partie d'une articulation artificielle,
- création de données de forme de l'implant ainsi configuré, et
- fabrication de l'implant sur la base des données de forme.

12. Procédé selon la revendication 11, dans lequel au moins un élément profilé et/ou un élément de plaque présentent une courbure tridimensionnelle.

13. Procédé selon la revendication 11 ou 12, dans lequel la structure de cadre (20, 25), la portion d'articulation (10) et/ou l'au moins un élément de plaque (51, 52) sont connectés par le biais d'une connexion soudée.

14. Procédé selon l'une quelconque des revendications 11 à 13, dans lequel au moins une portion de l'implant est fabriquée au moyen d'un procédé de fabrication additif.

15. Procédé selon l'une quelconque des revendications 11 à 14, dans lequel un élément de plaque, la portion d'articulation et/ou la structure de cadre est ou sont au moins en partie pourvu (e) (s) d'un traitement de surface.
